# EUROPEAN PATENT SPECIFICATION

(11) **EP 4 159 124 B1**
(45) Date of publication and mention of the grant of the patent: **03.04.2024**
(21) Application number: 22194803.7
(22) Date of filing: 30.09.2020
(51) Int. Cl.: A61B 5/287, A61B 5/00, A61B 18/00, A61B 18/14

(54) **INTRALUMINAL REFERENCE ELECTRODE FOR CARDIOVASCULAR TREATMENT APPARATUS**
INTRALUMINALE REFERENZELEKTRODE FÜR KARDIOVASKULÄRE BEHANDLUNGSVORRICHTUNG
ÉLECTRODE DE RÉFÉRENCE INTRALUMINALE POUR APPAREIL DE TRAITEMENT CARDIOVASCULAIRE

(30) Priority: 29.05.2020 US 202063031955 P; 16.07.2020 US 202063052553 P; 23.09.2020 US 202017029890; 23.09.2020 US 202017029752
(43) Date of publication of application: 05.04.2023
(62) Divisional of application: 20199230.2
(73) Proprietor: Biosense Webster (Israel) Ltd., 2066717 Yokneam (IL)
(72) Inventor: VAN NIEKERK, Pieter Emmelius, Irvine, 92618 (US); MALINARIC, Jamie Lynn, Irvine, 92618 (US); FUENTES-ORTEGA, Cesar, Irvine, 92618 (US); SALAZAR, Henry, Irwindale, 91706 (US); TOBEY, Dustin R., Irwindale, 91706 (US); BASU, Shubhayu, Irwindale, 91706 (US); THALER, Sean D., Irwindale, 91706 (US); WONG, Joseph R., Irwindale, 91706 (US); GHIDOLI, Daniele, Irwindale, 91706 (US)
(74) Representative: Carpmaels & Ransford LLP

(56) References cited:
- US-A1- 2014 316 294
- US-A1- 2019 350 649
- US-A1- 2020 129 125

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., radiofrequency (RF) energy and/or electroporation), it may be possible to cease or modify the propagation of unwanted electrical signals from one portion of the heart to another. The ablation process may provide a barrier to unwanted electrical pathways by creating electrically insulative lesions or scar tissue.

In some procedures, a catheter with one or more electrodes can be used to provide ablation within the cardiovascular system. The catheter can be inserted into a major vein or artery (e.g., the femoral artery) and then advanced to position the electrodes within the heart or in a cardiovascular structure adjacent to the heart (e.g., the pulmonary vein). The electrodes can be placed in contact with cardiac tissue or other vascular tissue and then electrically activated to ablate the contacted tissue. In some cases, the electrodes can be bipolar. In some other cases, a monopolar electrode may be used in conjunction with a ground pad that is in contact with the patient.

Examples of ablation catheters are described in U.S. Pub. No. 2013/0030426, entitled "Integrated Ablation System using Catheter with Multiple Irrigation Lumens," published Jan. 31, 2013; U.S. Pat. No. 10,660,700 entitled "Irrigated Balloon Catheter with Flexible Circuit Electrode Assembly," issued May 26, 2020; U.S. Pub. No. 2018/0071017, entitled "Ablation Catheter with a Flexible Printed Circuit Board," published Mar. 15, 2018; U.S. Pub. No. 2018/0056038, entitled "Catheter with Bipole Electrode Spacer and Related Methods," published Mar. 1, 2018; U.S. Pub. No. 2018/0036078, entitled "Catheter with Soft Distal Tip for Mapping and Ablating Tubular Region," published Feb. 8, 2018; U.S. Pat. No. 8,956,353, entitled "Electrode Irrigation Using Micro-Jets," issued Feb. 17, 2015; and U.S. Pat. No. 9,801,585, entitled "Electrocardiogram Noise Reduction," issued Oct. 31, 2017, for each of which a copy is provided in the priority U.S. Provisional Patent Application 63/052,553.

Some catheter ablation procedures may be performed using electrophysiology (EP) mapping. Such EP mapping may include the use of sensing electrodes on a catheter (e.g., the same catheter that is used to perform the ablation). The sensing electrodes can be the same or different electrodes as those used to perform ablation. The sensing electrodes can monitor electrical signals within the cardiovascular system to pinpoint the location of aberrant conductive tissue sites that are responsible for the arrhythmia. Examples of an EP mapping system are described in U.S. Pat. No. 5,738,096, entitled "Cardiac Electromechanics," issued Apr. 14, 1998 of which a copy is provided in the priority U.S. Provisional Patent Application 63/052,553. Examples of EP mapping catheters are described in U.S. Pat. No. 9,907,480, entitled "Catheter Spine Assembly with Closely-Spaced Bipole Microelectrodes," issued Mar. 6, 2018; U.S. Pat. No. 10,130,422, entitled "Catheter with Soft Distal Tip for Mapping and Ablating Tubular Region," issued Nov. 20, 2018; and U.S. Pub. No. 2018/0056038, entitled "Catheter with Bipole Electrode Spacer and Related Methods," published Mar. 1, 2018, for each of which a copy is provided in the priority U.S. Provisional Patent Application 63/052,553.

EP mapping procedures may include the use of a reference electrode on a mapping catheter to sense electrical potentials in fluids near tissue. An example of such reference electrode is described in U.S. Pub. No. 2020/0038101, entitled "Unipolar reference electrode for electrophysiology mapping catheter," published Feb. 6, 2020, of which a copy is provided in the priority U.S. Provisional Patent Application 63/052,553.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO system by Biosense Webster, Inc. of Irvine, Calif. Examples of catheters that are configured for use with an IGS system are disclosed in U.S. Pat. No. 9,480,416, entitled "Signal Transmission Using Catheter Braid Wires," issued Nov. 1, 2016, of which a copy is provided in the priority U.S. Provisional Patent Application 63/052,553 and various other references that are cited herein and attached in the Appendix in the priority U.S. Provisional Patent Application 62/052,553.

### SUMMARY

Examples presented herein generally include systems and methods which can contact electrodes to cardiac tissue while sensing electrical potential from fluids near the tissue. The electrodes in contact with the tissue can be carried by an end effector. The end effector sensors can be configured to ablate and/or sense electrical potentials from the cardiac tissue in which they are in contact. A reference electrode can be insulated by a shaft connected to the end effector so that the reference electrode can sense the electrical potentials from the fluids near the tissue while being prevented, by the shaft geometry, from contacting the cardiac tissue. The reference electrode can further serve as an extension of an irrigation tube positioned to irrigate the treatment area.

An example apparatus can include an elongated shaft, an end effector, and a reference electrode. A proximal portion of the elongated shaft can be manipulated to position a distal portion of the elongated shaft into a heart of a patient. The end effector can be disposed near the distal portion of the elongated shaft and can include spines each carrying at least one spine electrode. The spine electrodes can be positioned and otherwise configured to contact cardiovascular tissue. The spine electrodes can further be configured to receive electrical potentials from the tissue. The reference electrode can include a tubular, electrically conductive inner surface and an outer surface. The reference electrode can be disposed in the elongated shaft such that the electrode does not protrude beyond the elongated shaft. The inner surface can receive electrical potentials from fluids so that the electrical potentials received by the reference electrode act as a referential signal for the electrical potentials of tissue received by the electrodes carried by the spines. The outer surface of the reference electrode can be substantially electrically isolated from the electrical potentials of the fluids by an electrically insulating cover.

The apparatus can further include a connector joining the elongated shaft to the end effector. The connector can include the electrically insulating cover which electrically isolates the outer surface of the reference electrode.

The reference electrode can have a distal end approximately coplanar or coaxial to a distal end of the connector. The connector can be a distal extension of the shaft such that the reference electrode is approximately coplanar or coaxial with a distal end of the shaft by virtue of being approximately coplanar or coaxial with the distal end of the connector.

The connector can be capable of contacting the cardiovascular tissue while the spine electrodes are in contact with the cardiovascular tissue and while the reference electrode is electrically insulated, by the connector, from the cardiovascular tissue.

The end effector can have an unconstrained configuration aligned with a longitudinal axis of the apparatus and a particular geometry in the unconstrained configuration. The connector can include a tubular outer surface with a diameter measured orthogonal to the longitudinal axis. The end effector can have a height measured orthogonal to the longitudinal axis and a width measured orthogonal to the height and longitudinal axis when the end effector is in the unconstrained configuration. The height of the end effector can measure approximately equal to or less than the diameter of the tubular outer surface of the connector, and the width of the end effector can measure greater than the diameter of the tubular outer surface of the connector.

The apparatus can further include an irrigation tube in fluidic communication with the inner surface of the reference electrode. The reference electrode can function as a distal extension of the irrigation tube. The irrigation tube can be positioned within a lumen of the reference electrode and/or around the outer surface of the reference electrode. In addition, or as an alternative to the outer surface being electrically insulated by the connector joining the elongated shaft to the end effector, the outer surface of the reference electrode can be electrically insulated by a portion of the irrigation tube positioned over the outer surface of the reference electrode.

The spine electrodes can be positioned to preferably be distributed in a radially asymmetric pattern on the cardiovascular tissue in relation to the reference electrode's position when the plurality of spine electrodes are in contact with the cardiovascular tissue.

The spine electrodes can be positioned to form a rectangular grid when the plurality of spine electrodes are contact with the cardiovascular tissue.

The end effector can have a particular geometry including three loop members. Each of the loop members can include at least one of the spines (which each carry electrode positioned to contact tissue). The loop members can be joined at a common distal vertex. When the end effector is unconstrained, the distal vertex is aligned with a longitudinal axis defined by the elongated shaft. Each of the three loop members can include a respective pair of ends affixed to the distal portion of the elongated shaft. The end effector can deflect away from the unconstrained configuration at an angle relative to the longitudinal axis to position the spine electrodes against a planar surface.

The connector can secure each of the respective pair of ends of the loop members to the distal portion of the elongated shaft.

The end effector can deflect at an angle relative to the longitudinal axis to position the spine electrodes against a planar area of the cardiovascular tissue.

The spine electrodes can receive electrical potentials from the cardiovascular tissue when in contact with the cardiovascular tissue.

The spine electrodes can ablate when in contact with the cardiovascular tissue.

An example method can include one or more of the following steps presented in no particular order. An end effector can be assembled such that the end effector carries at least one spine electrode thereon that is configured to contact cardiovascular tissue and receive electrical potentials from the tissue. A reference electrode can be affixed in relation to the end effector such that a tubular inner surface of the reference electrode is configured to receive electrical potentials from fluids so that the electrical potential from the fluids act as a referential signal for the electrical potentials received by the spine electrodes. An outer surface of the reference electrode can be electrically insulated such that the outer surface is electrically isolated from the electrical potentials from fluids approximate the cardiovascular tissue while the inner surface is capable of receiving the electrical potentials of the fluids. The end effector and reference electrode can be affixed to a distal portion of an elongated shaft, the elongated shaft configured to be manipulated at a proximal portion of the elongated shaft to position the distal portion into a heart of a patient.

The method can further include joining the elongated shaft to the end effector with a connector and electrically insulating the outer surface of the reference electrode with the connector. The method can further include joining the end effector with the connector and an outer surface area of the reference electrode partially exposed electrode and positioned without the possibility of touching tissue.

The method can further include affixing the reference electrode and the connector such that a distal end of the reference electrode and a distal end of the connector are approximately coplanar and coaxial to each other. The connector can be a distal extension of the shaft such that the reference electrode is approximately coplanar or coaxial to the distal end of the shaft by virtue of being approximately coplanar or coaxial to the distal end of the connector.

The method can further include joining the elongated shaft to the end effector such that the connector is capable of contacting the cardiovascular tissue while the spine electrode is in contact with the cardiovascular tissue and while the reference electrode is electrically insulated, by the connector, from the cardiovascular tissue.

The method can further include assembling the end effector such that the end effector has an unconstrained configuration, and such that, when in the unconstrained configuration, the end effector has a height that is approximately equal to or less than a diameter of a tubular outer surface of the connector and the end effector has a width measuring greater than the diameter of the tubular outer surface of the connector, the diameter and height being measured orthogonal to a longitudinal axis defined by the elongated shaft, and the width being measured orthogonal to the longitudinal axis and direction of measurement of the height.

The method can further include positioning an irrigation tube within the elongated shaft such that the irrigation tube is in fluidic communication with the inner surface of the reference electrode.

The method can further include affixing the reference electrode to the irrigation tube such that the reference electrode is a distal extension of the irrigation tube.

The method can further include arranging the spine electrodes in a rectangular grid. Alternatively, the method can further include arranging the spine electrodes in a non-rectangular grid.

The method can further include shaping a first loop member, a second loop member and a third loop member to each form a respective loop; coupling a respective pair of ends for each of the first loop member, the second loop member, and the third loop member to the distal portion of the elongated shaft; joining the first loop member, the second loop member, and the third loop member at a common distal vertex distal to the distal portion of the elongated shaft; and configuring the end effector to be capable of deflecting at an angle relative to a longitudinal axis defined by the elongated shaft to position the spine electrodes against a planar surface.

The method can further include securing each of the respective pairs of ends to the distal portion of the elongated shaft with a connector and electrically insulating the outer surface of the reference electrode with the connector.

The method can further include configuring the end effector to deflect at an angle relative to the longitudinal axis to position the spine electrodes against a planar area of the cardiovascular tissue.

Another example method can include one or more of the following steps presented in no particular order. Steps of this example method are combinable with steps of the previous example method. A distal portion of the elongated shaft, and an end effector extending distally from a distal portion of the elongated shaft, can be moved through a catheter to a heart. The end effector can be moved from a distal end of the catheter via manipulation of a proximal portion of the elongated shaft. Spine electrodes, carried by the end effector, can be juxtaposed to cardiovascular tissue via manipulation of the proximal portion of the elongated shaft. While the spine electrodes are opposed to the cardiovascular tissue, electrical potentials from fluids approximate the cardiovascular tissue can be received via an inner surface of a reference electrode, an outer surface of the reference electrode being electrically insulated from the electrical potentials.

The method can further include contacting, to the cardiovascular tissue, while the spine electrodes are opposed (i.e., in physical contact) to the cardiovascular tissue, a connector joining the end effector to the distal portion of the elongated shaft and electrically insulating the outer surface of the reference electrode from the electrical potentials.

The method can further include irrigating the cardiovascular tissue through a lumen defined by the inner surface of the reference electrode.

The method can further include positioning the spine electrodes in a pattern on the cardiovascular tissue that is radially asymmetric in relation to the reference electrode's position.

The method can further include positioning the spine electrodes in a rectangular grid on the cardiovascular tissue.

The method can further include deflecting the end effector at an angle relative to a longitudinal axis defined by the elongated shaft to position the spine electrodes against the cardiovascular tissue. The cardiovascular tissue against which the spine electrodes are positioned being can be substantially planar.

The method can further include receiving, while the spine electrodes are opposed to the cardiovascular tissue, electrical potentials from the cardiovascular tissue via the spine electrodes.

The method can further include ablating the cardiovascular tissue via the spine electrodes, while the spine electrodes are opposed to the cardiovascular tissue. BRIEF

### DESCRIPTION OF THE DRAWINGS

Figure 1 is an illustration of a cardiovascular treatment apparatus having an end effector at a distal portion of an elongated shaft and a proximal handle at a proximal portion of the elongated shaft according to aspects of the present invention.
Figures 2A and 2B are illustrations of cross sectional views of an intermediate section and distal portion of a shaft of the apparatus in greater detail according to aspects of the present invention.
Figures 3A through 3C are various views of the end effector and distal portion of the shaft according to aspects of the present invention.
Figures 3D through 3F are illustrations of various views of the distal portion of the shaft according to aspects of the present invention.
Figures 4A through 4C are illustrations of the end effector being pressed to a planar surface according to aspects of the present invention.
Figures 5A and 5B are illustrations of cross sectional views of alternative reference electrode configurations according to aspects of the present invention.
Figure 6 is an illustration of a treatment utilizing the cardiovascular treatment apparatus according to aspects of the present invention.
Figures 7A through 7F are illustrations of alternative end effectors having loop members and including an intralumenal reference electrode according to aspects of the present invention.
Figure 8 is an illustration of an alternative end effector having a ray geometry and including an intralumenal reference electrode according to aspects of the present invention.
Figure 9 is an illustration of another alternative end effector including an intralumenal reference electrode according to aspects of the present invention.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the pertinent art from the following description, which includes by way of illustration, one of the best modes contemplated for carrying out the invention. As will be realized, the invention is capable of other different or equivalent aspects, all without departing from the invention as defined by the appended claims. Accordingly, the drawings and descriptions should be regarded as illustrative in nature and not restrictive.

Teachings, expressions, versions, examples, etc. described herein may be combined with other teachings, expressions, versions, examples, etc. that are described herein, including those examples provided in the references attached in the Appendix hereto. The following-described teachings, expressions, versions, examples, etc. should therefore not be viewed in isolation relative to each other. Various suitable ways in which the teachings herein may be combined are apparent to those skilled in the pertinent art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

As used herein, the term "non-transitory computer-readable media" includes, but is not limited to, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable information.

As used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

As used herein, the term "wire" can include elongated solid core and hollow core structures. When used to refer to an electrical conductor, the term wire "wire" can include insulated, non-insulated, individual, bundled, and integrated circuit conductors.

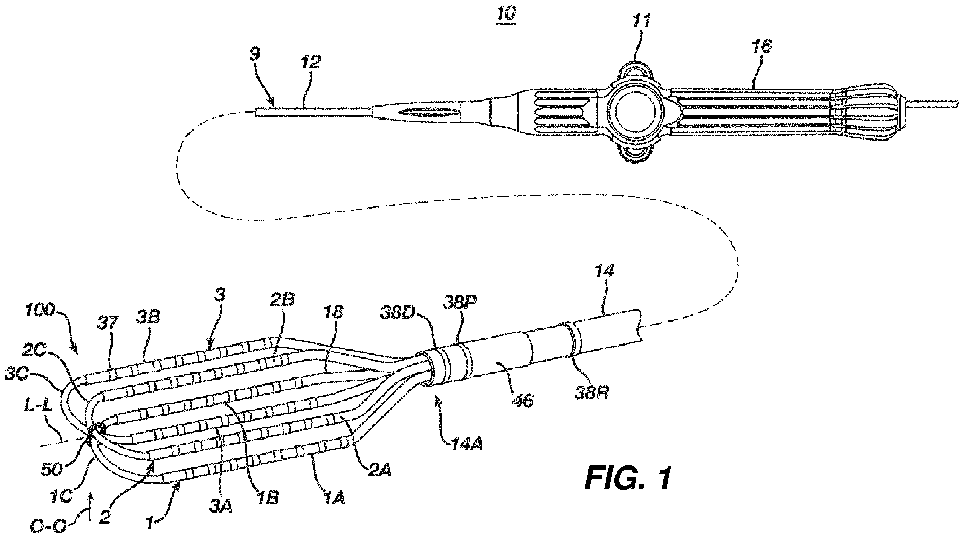

Figure 1 illustrates an example apparatus 10 having an elongated shaft 9, a distal electrode assembly or end effector 100, and a deflection control handle 16. The apparatus 10 can have several design variations while including novel aspects illustrated herein. The apparatus 10 is presented for illustration purposes only and is not intended to be limiting. Details of the apparatus can be understood with reference to U.S. Patent Application S.N. 63/031,955 to which the present application claims the benefits of priority.

The elongated shaft 9 has a proximal portion 12 in the shape of an elongated catheter body, an intermediate deflection section 14, and distal portion 14A. The deflection control handle 16 is attached to the proximal end of the catheter body 12. The distal portion 14A of the shaft is coupled to the end effector 100 via a connector tubing 46. The connector tubing 46 is considered herein to be a distal extension of the elongated shaft such that the connector tubing 46 is considered a part of the elongated shaft 9. The elongated shaft 9 forms a tubular catheter body sized and otherwise configured to traverse vasculature. The proximal portion 12 of the elongated shaft 9 can be manipulated (e.g. via the handle 16) to position the distal portion 14A of the shaft 9 into a heart of a patient. The end effector 100 has a plurality of loop members 1, 2, 3 that overlap at a common distal vertex 50. The loop members 1, 2, 3 can be joined at the distal vertex 50 by a mechanical linkage.

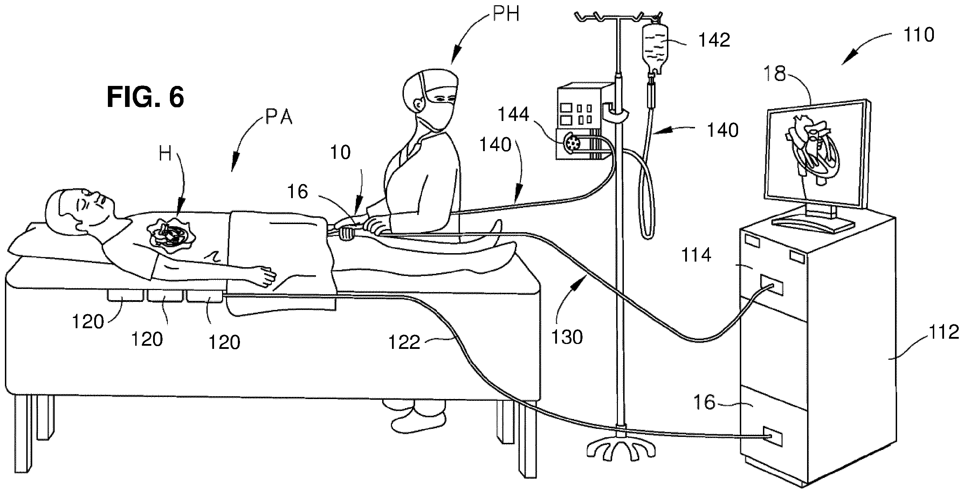

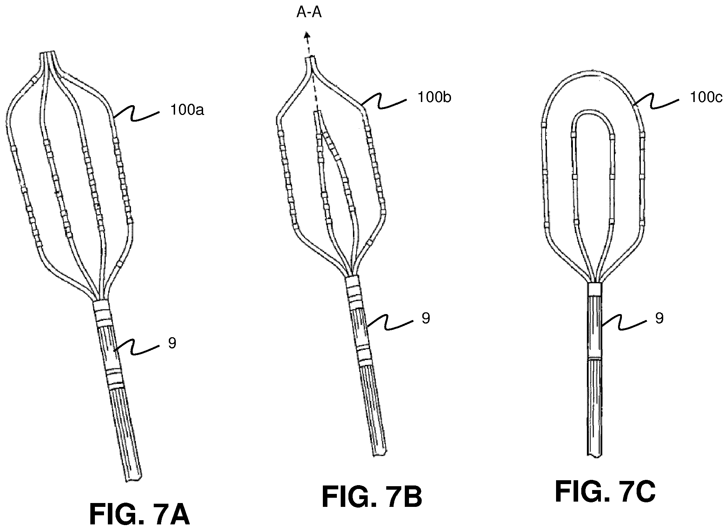

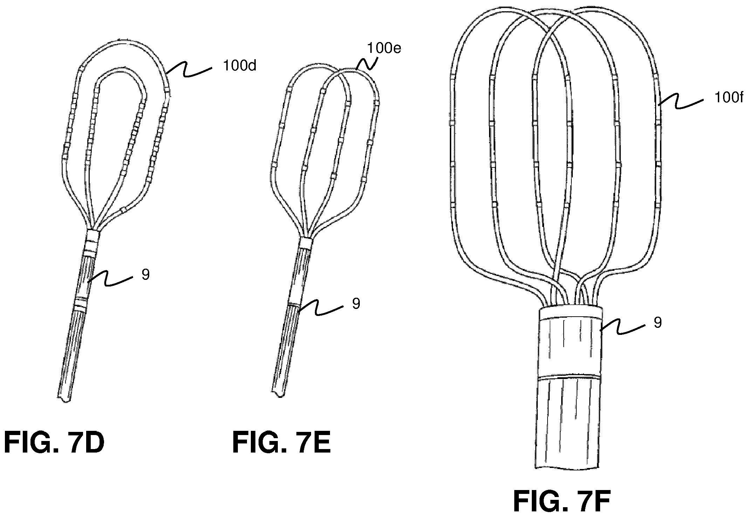

The end effector 100 is illustrated in an unconstrained configuration. When the device is unconstrained and aligned, the proximal portion 12, intermediate section 14, distal portion 14A, and end effector 100 are generally aligned along a longitudinal axis A-A. The elongated shaft 9 can define the longitudinal axis A-A of the apparatus 10. The intermediate section 14 can be configured to bend to deflect the distal portion 14A and end effector 100 from the longitudinal axis A-A similar to as described in U.S. Pat. No. 9,820,664 (see Figures 6 and 7). Details of the apparatus can be understood with reference to U.S. Patent Application S.N. 63/031,955.

The end effector 100 can be collapsed (compressed toward the longitudinal axis A-A) to fit within a guiding sheath or catheter (not illustrated). The shaft 9 can be pushed distally to move the end effector 100 distally through the guiding sheath. The end effector 100 can be moved to exit a distal end of the guiding sheath via manipulation of the shaft 9 and/or control handle 16. An example of a suitable guiding sheath for this purpose is the Preface Braided Guiding Sheath, commercially available from Biosense Webster, Inc. (Irvine, California, USA).

The end effector 100 has first, second and third loop members 1, 2, and 3. Each loop member 1, 2, 3 has two spines 1A, 1B, 2A, 2B, 3A, 3B and a connector 1C, 2C, 3C that connects the two spines of the respective loop member 1, 2, 3. Spines 1A, 1B of the first loop member 1 are connected by a first connector 1C; spines 2A, 2B of the second loop member 2 are connected by a second connector 2C; and spines 3A, 3B of the third loop member 3 are connected by a third connector 3C. Each loop member 1, 2, 3 further has a respective pair of ends affixed to the distal portion 14A of the elongated shaft 9 (e.g. affixed to the connector 46 which is an extension of the distal portion 14A of the elongated shaft 9).

For each loop member 1, 2, 3 the spines 1A, 1B, 2A, 2B, 3A, 3B in the respective pair of spines can be substantially parallel to each other along a majority of their respective lengths when the end effector 100 is expanded in an unconstrained configuration as illustrated in Figure 1. Preferably, all spines in the end effector are parallel to each other along the majority of their respective lengths when the end effector 100 is in the unconstrained configuration. Even when all spines are parallel, the spines are not necessarily all coplanar. Details of the apparatus can be understood with reference to U.S. Patent Application S.N. 63/031,955.

Each spine 1A, 1B, 2A, 2B, 3A or 3B can have a length ranging between about 5 and 50 mm, preferably between about 10 and 35 mm, and more preferably about 28 mm. The parallel portions of each spine 1A, 1B, 2A, 2B, 3A, 3B can be spaced apart from each other by a distance ranging between about 1mm and 20 mm, preferably between about 2 and 10 mm, and more preferably about 4 mm. Each spine 1A, 1A, 1B, 2A, 2B, 3A, 3B preferably carries at least eight electrodes per spine member. The end effector preferably includes six spines as illustrated. With eight electrodes on six spines, the end effector 100 includes forty-eight electrodes. The spine electrodes 37 can be positioned and otherwise configured to contact cardiovascular tissue. The spine electrodes 37 can be configured to receive electrical potentials from tissue that they are in contact with and/or ablate the tissue.

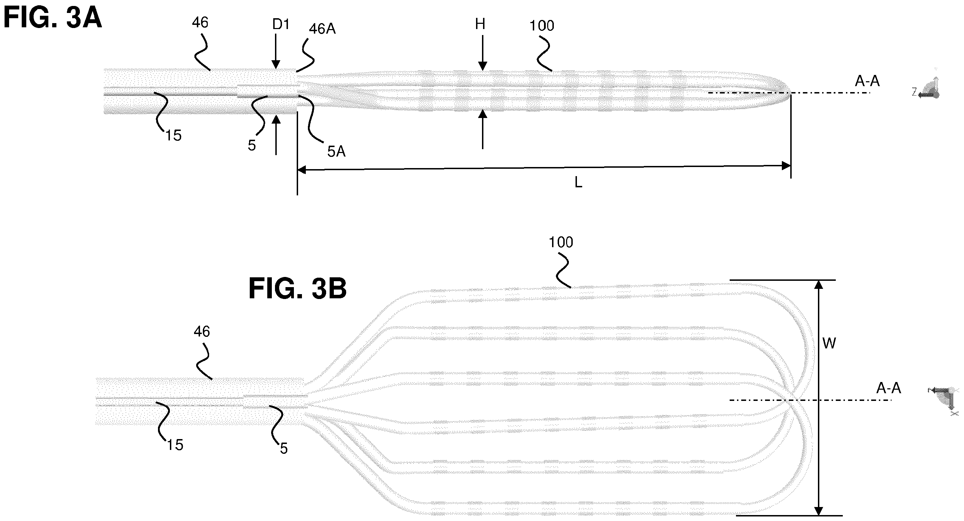

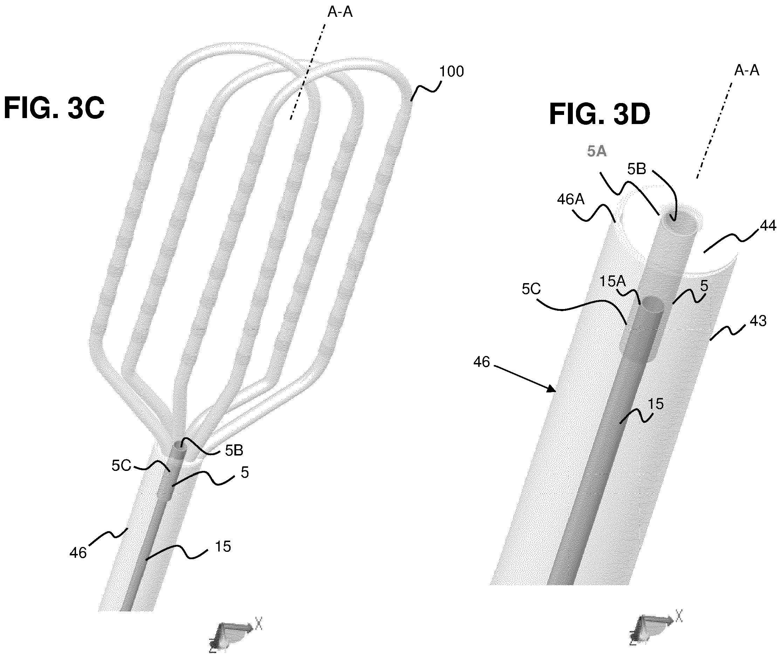

Figure 1 is illustrated as including a distal electrode 38D and a proximal electrode 38P positioned near the distal portion 14A of the shaft 9. The electrodes 38D and 38P can be configured to cooperate (e.g. by masking of a portion of one electrode and masking a different portion on the other electrode) to define a referential electrode (an electrode that is not in contact with tissues). As illustrated in greater detail in Figures 3A through 3F, the apparatus 10 can include a reference electrode 5 positioned within the connector tubing 46 so that is it is prevented from contacting tissue by the connector tubing 46. The reference electrode 5 can be used in addition to, or in place of one or both of the distal electrode 38D and the proximal electrode 38P illustrated in Figure 1.Due to the likelihood of the electrodes 38D and 38P coming into contact with tissues, it is preferable that reference electrode 5 is utilized by itself.

In some unipolar EP mapping techniques, it can be desirable to obtain a reference potential from blood near the tissue at which a tissue potential is being picked up. In other words, it may be desirable to place a first electrode in contact with tissue to thereby pick up an electrical potential from the tissue; and place a second electrode in electrical communication with blood near the contacted tissue to thereby pick up a reference electrical potential from the blood. The reference electrode can be in direct contact with the blood and/or be in electrical communication with the blood via other fluids such as irrigation fluid. The second (reference) electrode can be configured such that it is prevented from contacting tissue while maintaining electrical communication with blood. By having the second (reference) electrode avoid contact with tissue, the second (reference) electrode may avoid pickup of local tissue potentials that might otherwise compromise the reliability of the sensed reference potential. This configuration can provide benefits similar to those obtained using bipolar EP mapping devices and techniques, such as reduced noise and reduced far field signals, due to the location of the reference electrode being in the same heart chamber as tissue-contacting electrodes; while still maintaining features of a unipolar signal.

Referring again to Figure 1, it is expected that when the end effector 100 is pressed to tissue, one side of each of the reference electrodes 38D, 38P on the outside shaft 9 may contact tissue while the opposite side of each of the shaft reference electrodes 38D, 38P faces away from the tissue. The electrodes 38D, 38P can be masked with an electrical insulator on opposite sides such that when an unmasked side of one of the electrodes is against tissue, an unmasked side of the other electrode is facing away from the tissue; meaning, if one of the shaft reference electrodes 38D, 38P is in electrical contact with tissue, the other of the shaft electrodes 38D, 38P is insulated from the tissue by an electrically insulating mask.

Examples presented herein include a reference electrode 5 that has an electrically insulated outer surface such that electrically conductive portions of the reference electrode 5 are prevented from ever coming into contact with tissue. This eliminates the need for two electrodes (e.g. aforementioned shaft electrodes 38D, 38P) thereby reducing wires and bulk. The end effector 100 can be joined to the connector tubing 46 such that an outer surface area of the reference electrode 5 is partially exposed electrically and the exposed outer surface is positioned to prohibit the possibility that the exposed outer surface touches tissue during treatment.

One or more impedance sensing electrodes 38R can be configured to allow for location sensing via impedance location sensing technique, as described in U.S. Pat. Nos. 5,944,022; 5,983,126; and 6,445,864, for each of which a copy is provided in the priority U.S. Provisional Patent Application 63/052,553.

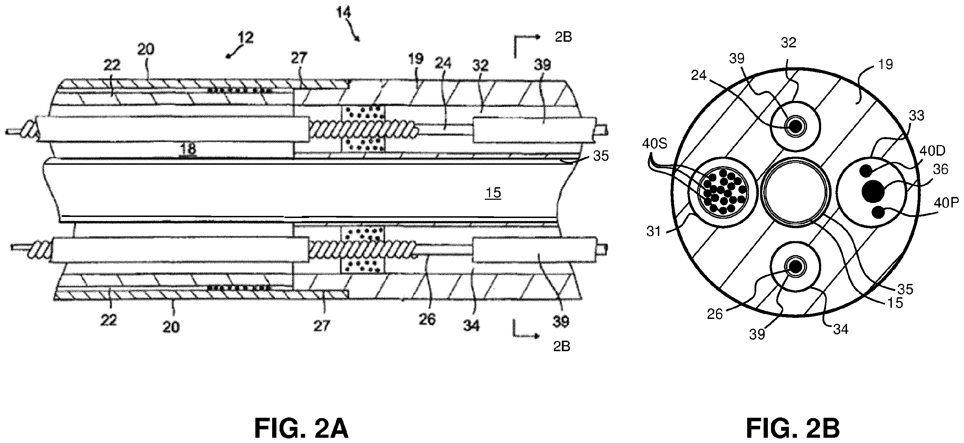

Figures 2A and 2B illustrate the intermediate section 14 and distal portion 14A of the shaft 9 of the apparatus in greater detail. Figure 2A is a cross-sectional view, along the longitudinal axis A-A, of the elongated shaft 9 at the interface between the proximal portion 12 and intermediate section 14. Figure 2B is a cross-sectional view of the intermediate section 14 orthogonal to the longitudinal axis A-A.

As illustrated in Figure 2A, the catheter body 12 can be an elongated tubular construction having a single axial passage or central lumen 18. The central lumen 18 can be sized to allow an irrigation tube 15 to pass therethrough. The catheter body 12 is flexible, i.e., bendable, but substantially non-compressible along its length. The catheter body 12 can be of any suitable construction and made of any suitable material. In some embodiments, the catheter body 12 has an outer wall 20 made of polyurethane or PEBAX. The outer wall 20 may include an imbedded braided mesh of stainless steel or the like to increase torsional stiffness of the catheter body 12 so that, when the control handle 16 is rotated, the intermediate section 14 will rotate in a corresponding manner.

The outer diameter of the catheter body 12 is preferably no more than about 8 French, more preferably about 7 French. The thickness of the outer wall 20 is thin enough so that the central lumen 18 can accommodate at least one puller wire, one or more lead wires, and any other desired wires, cables or tubes (e.g. irrigation tube 15). If desired, the inner surface of the outer wall 20 is lined with a stiffening tube 22 to provide improved torsional stability. In some embodiments, the outer wall 20 has an outer diameter of from about 0.090 inch to about 0.094 inch (from about 2.3 mm to about 2.4 mm) and an inner diameter of from about 0.061 inch to about 0.065 inch (from about 1.5 mm to about 1.7 mm).

As illustrated particularly in Figure 2B, the intermediate section 14 can include a shorter section of tubing 19 having multiple lumens, for example, four off-axis lumens 31, 32, 33, 34 and a central lumen 35. The first lumen 31 carries a plurality of lead wires 40S for ring electrodes 37 carried on the spines 1A, 1B, 2A, 2B, 3A, 3B. The second lumen 32 carries a first puller wire 24. The third lumen 33 carries a cable 36 for an electromagnetic position sensor 42 and lead wires 40D and 40P for distal and proximal ring electrodes 38D and 38P carried on the catheter proximally of the end effector 100 and/or the reference electrode 5 within the tubing 46.

Electromagnetic location sensing technique is described in U.S. Patent Nos. 5,391,199; 5,443,489; 5,558,091; 6,172,499; 6,590,963; and 6,788,967, for each of which a copy is provided in the priority U.S. Provisional Patent Application 63/052,553. The magnetic location sensor 42 can be utilized with impedance sensing electrode 38R in a hybrid magnetic and impedance position sensing technique known as ACL described in US Patent Nos. 7,536,218; 7,756,567; 7,848,787; 7,869,865; and 8,456,182, for each of which a copy is provided in the priority U.S. Provisional Patent Application 63/052,553.

The fourth lumen 34 (for example, diametrically opposite of the second lumen 32 as illustrated) carries a second puller wire 26. The fifth, central lumen 35 carries the irrigation tube 15.

The tubing 19 is made of a suitable non-toxic material that is preferably more flexible than the catheter body 12. One suitable material for the tubing 19 is braided polyurethane, i.e., polyurethane with an embedded mesh of braided stainless steel or the like. The size of each lumen is sufficient to house the lead wires, puller wires, the cable and any other components.

The useful length of the catheter shaft 9, i.e., that portion of the apparatus 10 that can be inserted into the body excluding the end effector 100, can vary as desired. Preferably the useful length ranges from about 110 cm to about 120 cm. The length of the intermediate section 14 (measured from the connection to the catheter body 12 to the distal end of the shaft 9) is a relatively smaller portion of the useful length, and preferably ranges from about 3.5 cm to about 10 cm, more preferably from about 5 cm to about 6.5 cm.

Catheter body proximal portion 12 can be attached to the intermediate section 14 similar to as shown and described in Figures 2A and 2B of U.S. Pat. No. 9,820,664, of which a copy is provided in the priority U.S. Provisional Patent Application 63/052,553 If desired, a spacer (not shown) can be located within the catheter body 12 between the distal end of the stiffening tube (if provided) and the proximal end of the intermediate section 14. The spacer can provide a transition in flexibility at the junction of the catheter body 12 and intermediate section 14, which can allow this junction to bend smoothly without folding or kinking. A catheter having such a spacer is described in U.S. Pat. No. 5,964,757, of which a copy is provided in the priority U.S. Provisional Patent Application 63/052,553.

The distal portion 14A of the shaft 9 can be substantially contiguous with the intermediate section 14 such that the intermediate section comprises the distal portion 14A; the distal portion being distinguished from the intermediate section 14 by the positioning of one or more (optional) ring electrodes 38R. As referred to herein, the distal portion 14A of the shaft 9 can therefore correspond to a distal portion of the intermediate section 14. The connector tubing 46 can also be considered a distal extension of the shaft 9 so that it is understood that the connector tubing 46 is included in the distal portion 14A of the shaft.

Figures 3A through 3C illustrate the end effector and distal portion of the shaft 9 (connector tubing 46) in various orientations where the connector tubing is illustrated as translucent and all components within the connector tubing are made invisible with the exception of the reference electrode 5 and the irrigation tube 15 for the purposes of illustration. The connector tubing 46 can further house ends of support frames which extend through the loop members 1, 2, 3 to secure the loop members 1, 2, 3 to the shaft 9 similar to the apparatus described and illustrated in U.S. Patent Application S.N. 63/031,955. The connector tubing 46 can further house electrical conductors 36, 40D, 40P, 40S such as those illustrated in Figures 2A and 2B.

Figures 3D through 3F illustrate the connector tubing in various orientations with the end effector 100 made invisible for the purpose of illustration.

A Cartesian x, y, z axis is illustrated in each of the Figures 3A through 3F to illustrate relative orientations for each of the illustrations.

Referring collectively to Figures 3A through 3F, the reference electrode 5 can include a tubular inner surface 5B and a tubular outer surface 5C. The inner tubular surface 5B can be electrically conductive. The inner surface 5B can be positioned and otherwise configured so that fluids near cardiovascular tissue (e.g. blood and/or irrigation fluid) are in contact with the inner surface 5B when the spine electrodes 37 are in contact with cardiovascular tissue. The inner surface 5B can receive electrical potentials from these fluids so that the electrical potentials received by the reference electrode act as a referential signal for the electrical potentials of the electrodes 37 carried by the spines 1A, 1B, 2A, 2B, 3A, 3B.

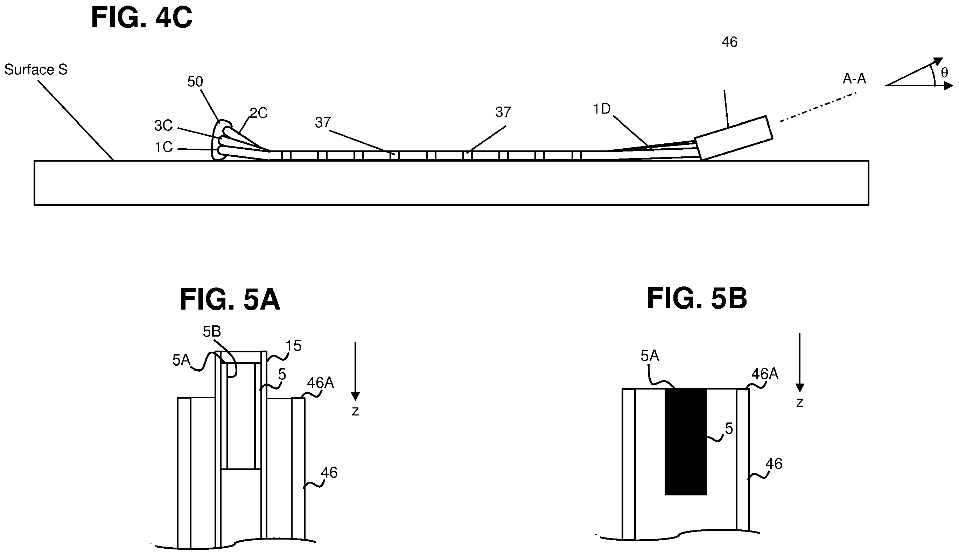

The reference electrode 5 can be disposed in the elongated shaft 9 (being disposed in the connector 46 which is a distal extension of the shaft 9) such that the electrode does not protrude beyond the elongated shaft 9. The outer surface 5C of the reference electrode 5 can be substantially electrically isolated from the electrical potentials of the fluids. The connector 46 of the shaft 9 can serve as an insulating cover which electrically isolates the outer surface 5C. The reference electrode 5 can have a distal end 5A that is approximately coaxial or coplanar with a distal end 46A of the connector 46 (which corresponds to a distal end of the shaft 9). The term "coplanar" as used in relation to the reference electrode distal end 5A and the distal end 46A means that a plane may intersect both 5A and 46A so that both ends (5A and 46A) are on the same plane. In the example shown in Fig. 3D, a virtual plane (not shown) intersects surface 5C and 46A orthogonally so that both ends 5A and 46A are on the same plane. The term "coaxial" indicates that the longitudinal axis of connector 46 and the longitudinal axis of the reference electrode 5 are coincident with each other. The connector 46 can be capable of contacting the cardiovascular tissue while the spine electrodes are in contact with the cardiovascular tissue and while the reference electrode 5 is electrically insulated, by the connector, from the cardiovascular tissue as illustrated in greater detail in Figure 4C.

The irrigation tube can be in fluidic communication with the inner surface 5B of the reference electrode 5. Fluids can flow through the irrigation tube and out of the shaft 9 through the reference electrode 5 so that the reference electrode 5 serves as a distal extension of the irrigation tube 15.

As illustrated in Figures 3A and 3B, the end effector 100 can have an unconstrained configuration aligned with a longitudinal axis A-A of the apparatus 10 and a flattened or paddle shaped geometry in the unconstrained configuration. The end effector 100 can have a height H and a width W measured as indicated in Figures 3A and 3B, the height H being significantly smaller than the width W. Further, compared to the dimensions of the connector 46, the height H of the end effector 100 can be about equal to, or smaller than, a diameter D1 of a tubular outer surface of the connector 46, where the diameter D1 is measured as indicated in Figure 3A. The width W of the end effector 100 can be substantially greater than the diameter D1 of the connector 46. The end effector can have a length L measured as indicated in Figure 3A that is greater still than the width W.

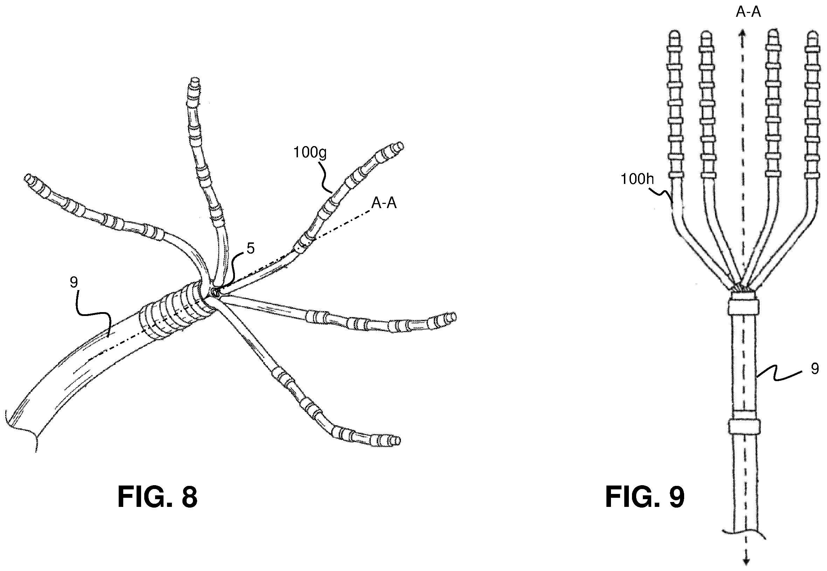

Having the paddle shape, the spine electrodes 37 are all positioned in a distal direction in relation to the reference electrode 5. When pressed to a surface, the spine electrodes 37 and references electrode 5 can roughly maintain their relative positions to each other compared to the unconstrained configuration. This results in the spine electrodes 37 being positioned to preferably be distributed in a radially asymmetric pattern on the cardiovascular tissue in relation to the reference electrode's position when the plurality of spine electrodes 37 are in contact with the cardiovascular tissue. In other words, the reference electrode 5 is positioned to one side of the collection of spine electrodes 37 when the electrodes 5, 37 are in operation during a treatment. This in contrast to other end effector geometries, such as illustrated in Figure 8 where spines of the end effector extend radially from the reference electrode 5 so that it is possible to preferably distribute spine electrodes in a radially symmetric pattern on the cardiovascular tissue in relation to the reference electrode's position. Paddle shaped end effectors such as illustrated in Figures 7A through 7F also include spine electrodes positioned to be distributed in a radially asymmetric pattern on tissue in relation to the reference electrode's position.

The spine electrodes 37 can be positioned to form a rectangular grid when the spine electrodes 37 are contact with the cardiovascular tissue. Alternatively, the spine electrodes 37 can be positioned to form a non-rectangular grid; e.g. a circular, triangular, or other such shape.

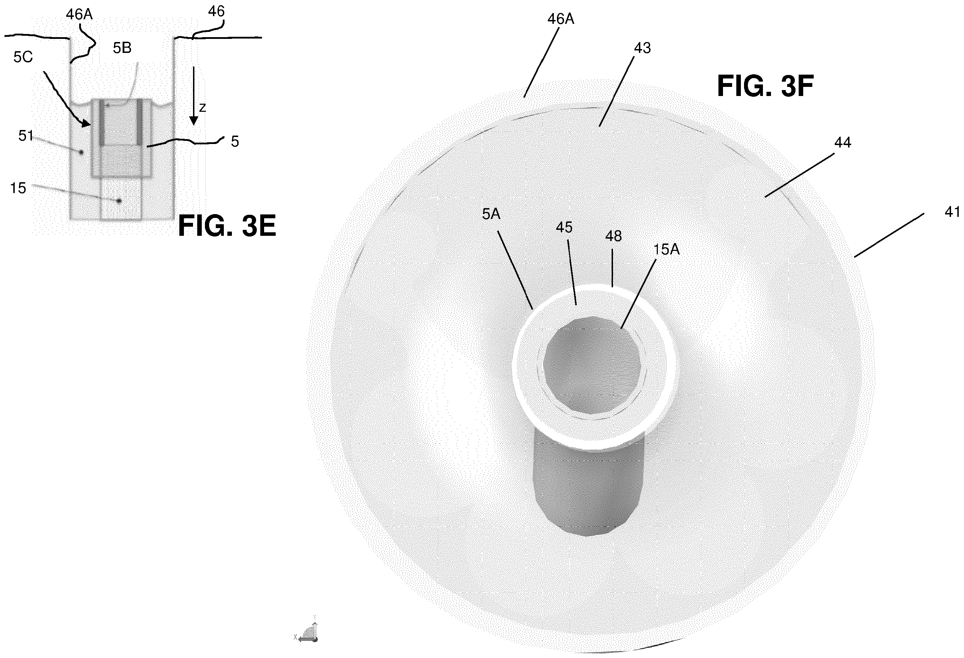

As illustrated in greater detail in Figures 3D and 3E, the irrigation tube 15 can be positioned within a lumen of the reference 5 electrode. The apparatus 10 can further include an irrigation tube cover 45 to act as a fluid impermeable seal between the reference electrode 5 and the irrigation tube 15.

The distal end 5A of the electrode 5 is approximately co-planar with the distal end 46A of the connector 46 or even recessed (Fig. 3E) so that the electrode 5 does not protrude from the shaft 9 (e.g., Fig. 1). An insulating material 51 can be provided between the outer surface 5C of the electrode 5 and the inside surface 46B of connector 46. In one embodiment, material 51 can be a polymer such as for example, polyurethane. Configured as such, insulating material 51 insulates the outer surface 5C of the electrode 5 to prevent the outer surface 5C from coming into electrical contact with tissue during use. At the same time, however, inner surface 5C of reference electrode 5 is able to have electrical and physical contact with the biological fluid in the organ (e.g., blood) in order to receive or record the electrical signals propagated in the fluid. It is noted that irrigation line 15 can also work in reverse, by aspirating or sucking blood from the organ into the irrigation line 15. This allows for the reference electrode 5 to receive or sense electrical signals (via wiring 5D or electrical trace) in the blood significantly better because the blood is pulled into irrigation tube 15 allowing immersion of the conductive inner surface 5C with the blood pulled into irrigation tube 15.

As illustrated in greater detail in Figure 3F, the connector 46 can include a collar 41 forming the outer surface 46A of the connector 46 and an insert 43 positioned in the collar 41. The insert 43 can include openings 44 shaped to receive ends of the loop members 1, 2, 3 and a central lumen 48 sized to house the reference electrode 45. A polymer can be flowed into the collar 41 and insert 43 to help secure the loop members 1, 2, 3 and reference electrode 5 in the connector 46.

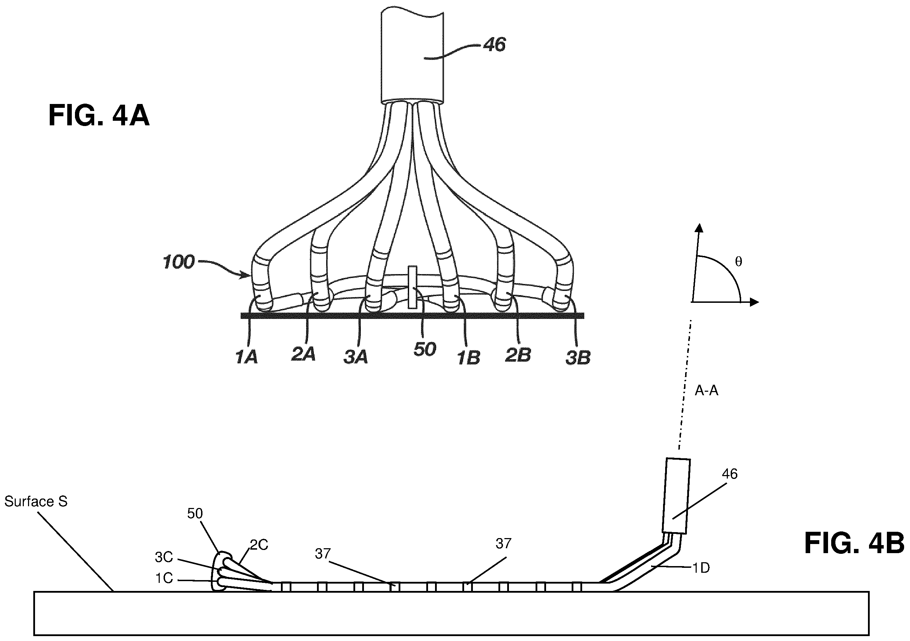

Figures 4A through 4C are illustrations of the end effector 100 being pressed to a surface S. The end effector 100 can deflect at an angle θ relative to the longitudinal axis A-A to position the spine electrodes 37 to the surface S. The surface S is illustrated as planar; however, the surface can be curved. For instance, the surface S can have curvature consistent with intracardiac tissue surfaces.

Figure 4A illustrates the end effector 100 having a portion of spine electrodes 37 pressed to the surface S and another portion of the spine electrodes 37 above the surface S, not in contact with the surface S.

Figure 4B illustrates the connector 46 positioned above the surface S with all spine electrodes 37 in contact with the surface. The end effector 100 is deflected an angle θ relative to the longitudinal axis A-A that is nearly 90°. At such an angle, were the reference electrode 5 positioned such that it protruded from the shaft 9, depending on the length of protrusion and the distance between the distal end 46A of the connector 46 and the surface S, the reference electrode 5 may come into contact with the surface S. If the reference electrode 5 were to make electrical contact with the surface S, it can reduce the effectiveness of the reference electrode's ability to sense electrical potentials that can serve as reference electrical potentials. In other words, were the reference electrode 5 to come into electrical contact with tissue, it may not function as a reliable reference electrode. Positioning the reference electrode 5 so that it does not protrude from the connector 46 of the shaft 9 prevents the reference electrode 5 from contacting the surface S even when the end effector 100 is deflected at an angle of about 90° from the longitudinal axis A-A of the shaft 9.

Figure 4C illustrates the connector 46 in contact with the surface S with all spine electrodes 37 in contact with the surface S. The end effector 100 is deflected at an acute angle θ relative to the longitudinal axis A-A. Having the connector 46 very near to the surface S and at a non-zero angle to the surface S, were the reference electrode 5 positioned such that it protruded from the shaft 9, depending on the length of protrusion and the angle θ, the reference electrode 5 may come into contact with the surface S. Positioning the reference electrode 5 so that it does not protrude from the connector 46 of the shaft 9 prevents the reference electrode 5 from contacting the surface S even when the connector 46 is in contact with the surface S and angled toward the surface S.

Figures 5A and 5B are illustrations of an alternative reference electrode configuration. The figures include a z axis to provide orientation relative to Figures 3A through 3F.

Figure 5A is an illustration of a cross section of an alternative reference electrode configuration. Only the connector 46, reference electrode 5, and irrigation tube 5 are illustrated for the purposes of illustration. The apparatus 10 can include additional components not illustrated such as conductors and mechanical connection to the end effector 100 described elsewhere herein, including the Appendix. The irrigation tube 15 can be positioned around the outer surface 5C of the reference electrode 5. The transition from the irrigation tube 15 to the reference electrode 5 can be sealed to cause the reference electrode 5 to be an extension of the irrigation tube 15. In addition, or as an alternative to the outer surface 5C being electrically insulated by the connector 46, the outer surface 5C of the reference electrode 5 can be electrically insulated by a portion of the irrigation tube 15 positioned over the outer surface 5C of the reference electrode 5. In some configurations, such as illustrated, the irrigation tube 15 and reference electrode 5 can protrude from the elongated shaft so that the irrigation tube 15 prevents the outer surface 5C of the reference electrode 5 from coming into contact with tissue. For instance, the distal end 5A of the reference electrode 5 can be positioned in the irrigation tube 15 so that the reference electrode 5 does not protrude from the irrigation tube 15.

Figure 5B is an illustration of a cross section of another alternative reference electrode configuration. In the alternative configuration, the reference electrode does not serve as an extension to the irrigation tube 15 but rather has an electrically conductive distal surface at its distal end 5A that is configured to receive electrical potentials from fluids that act as a reference signal for the electrical potentials from the spine electrodes 37. The electrically conductive surface at the distal end 5A of the reference electrode 5 is approximately coplanar or coaxial to the distal end 46A of the connector 46. Only the connector 46 and reference electrode 5 are illustrated for the purposes of illustration. The apparatus 10 can include additional components not illustrated such as conductors and mechanical connection to the end effector 100 described elsewhere herein, including in the Appendix.

Figure 6 is an illustration of an exemplary medical procedure and associated components of a cardiac electrophysiology (EP) mapping catheter system that may utilize the apparatus 10 (also referred to as a catheter assembly). A physician PH is illustrated grasping the handle 16 of the apparatus, with the end effector 100 (not shown in Figure 6) disposed in a patient PA to perform EP mapping in or near the heart H of the patient PA. The apparatus 10 is coupled with a guidance and drive system 110 via a cable 130. The apparatus 10 can optionally be coupled with a fluid source 142 via a fluid conduit 140. A set of field generators 120 are positioned underneath the patient PA and are coupled with guidance and drive system 110 via another cable 122. Field generators 120 are also optional.

The guidance and drive system 110 can include a console 112 and a display 118. The console 112 can include a first driver module 114 and a second driver module 116. The first driver module 114 can be coupled with the apparatus via a cable 130. In some variations, the first driver module 114 is operable to receive EP mapping signals obtained via spine electrodes 37 of end effector 100. The console 112 can include a processor (not shown) that processes such EP mapping signals and thereby provides EP mapping. In addition, or in the alternative, the first driver module 114 may be operable to provide RF power to the spine electrodes 37 of end effector 100 to thereby ablate tissue. In some versions, the first driver module 114 is also operable to receive position indicative signals from a position sensor in end effector 100. In such versions, the processor of console 112 is also operable to process the position indicative signals from the position sensor to thereby determine the position of the end effector 100 within the patient PA.

The guidance and drive system 110 can further include non-transitory computer readable medium with instructions thereon to case the drive system 110 to perform functionality described herein and/or as are known related to use of similar equipment. In some examples, the non-transitory computer readable memory can be in communication with the first driver module 114 (e.g. by virtue of being in communication with a processor of the first driver module 114 and/or the processor of the console 112). The non-transitory computer readable medium can include instructions thereon that when executed by the first driver module 114 cause the first driver module 114 to receive EP mapping signals from the spine electrodes 37 and a reference signal from the reference electrode 5.

The second driver module 116 is coupled with field generators 120 via a cable 122. The second driver module 116 is operable to activate field generators 120 to generate an alternating magnetic field around the heart H of the patient PA. For instance, the field generators 120 may include coils that generate alternating magnetic fields in a predetermined working volume that contains the heart H.

Some versions of the apparatus 10 include a position sense near or within the end effector 100 that is operable to generate signals that are indicative of the position and orientation of end effector 100 within the patient PA. Each position sensor may include a wire coil or a plurality of wire coils (e.g., three orthogonal coils) that are configured to generate electrical signals in response to the presence of an alternating electromagnetic field generated by field generators 120. Other components and techniques that may be used to generate real-time position data associated with end effector 100 may include wireless triangulation, acoustic tracking, optical tracking, inertial tracking, and the like. By way of example only, position sensing may be provided in accordance with at least some of the teachings of U.S. Pat. No. 9,480,416, of which a copy is provided in the priority U.S. Provisional Patent Application 63/052,553. Alternatively, apparatus 10 may lack a position sensor near the end effector 100.

The display 118 is coupled with the processor of console 112 and is operable to render images of patient anatomy. Such images may be based on a set of preoperatively or intraoperatively obtained images (e.g., a CT or MRI scan, 3-D map, etc.). The views of patient anatomy provided through the display 118 may also change dynamically based on signals from the position sensor near the end effector 100.

The processor of the console 112 may also drive the display 118 to superimpose the current location of end effector 100 on the images of the patient's anatomy, such as by superimposing an illuminated dot, a crosshair, a graphical representation of end effector 100, or some other form of visual indication.

The fluid source 142 can include a bag containing saline or some other suitable irrigation fluid. The conduit 140 can include a flexible tube that is further coupled with a pump 144, which is operable to selectively drive fluid from the fluid source 142 to the irrigation tube 15 of the apparatus 10. In some variations, such as including a reference electrode 5 as configured in Figure 5B, the conduit 140, fluid source 142, and pump 144 are omitted entirely.

Figures 7A through 7F are illustrations of alternative end effectors 100a-f having loop members and including an intralumenal reference electrode 5 described elsewhere herein. The end effectors include a reference electrode that does not protrude from the shaft 9 and therefore is not visible in the views of the figures. The end effectors can otherwise be configured similarly to the end effectors illustrated in U.S. Pat. No. 9,820,664 (see Figures 8A through 8F), U.S. Pub. No. 2020/0038101, and U.S. Pub. No. 2020/0038101 (see Figures 10 through 15), for each of which a copy is provided in the priority U.S. Provisional Patent Application 63/052,553.

Figure 8 is an illustration of an alternative end effector 100g having a ray geometry and including an intralumenal reference electrode according to aspects of the present invention. The end effector includes a reference electrode 5 that does not extend beyond the shaft 9. The end effector can otherwise be configured similarly to the end effectors illustrated in U.S. Pat. No. 9,907,480 (see Figure 5), U.S. Pub. No. 2018/0056038 (see Figure 5), and U.S. Pub. No. 2020/0038101 (see Figures 2 through 7, 19, and 20). It is noted that the reference electrode as described and illustrated herein can be utilized with an end effector having a basket configuration whereby each of the spines expand outward and converges towards a distal end is within the scope of the invention. One example of such basket configuration is shown exemplarily in U.S. Pat. No. 10,575,743B2, which is incorporated by reference herein.

Figure 9 is an illustration of another alternative end effector 100h including an intralumenal reference electrode according to aspects of the present invention. The end effectors include a reference electrode that does not protrude from the shaft 9 and therefore is not visible in the views of the figures. The end effector can otherwise be configured similarly to the end effectors illustrated in U.S. Pat. No. 9,820,664 (see Figures 5A through 7) and U.S. Pub. No. 2020/0038101 (see Figure 16).

## Claims

1. An apparatus comprising:
an elongated shaft (9) comprising a proximal portion (12) and a distal portion (14A), the elongated shaft configured to be manipulated at the proximal portion to position the distal portion into a heart of a patient;
an end effector (100) disposed proximate the distal portion of the elongated shaft and comprising spines (1A, 1B, 2A, 2B, 3A, 3B) each carrying at least one spine electrode (37) configured to contact cardiovascular tissue and receive electrical potentials from the tissue; **characterized in**
a reference electrode (5) comprising an electrically conductive distal surface at its distal end, the reference electrode being disposed in the elongated shaft,
the electrically conductive distal surface being configured to receive electrical potentials from fluids that act as a referential signal for the electrical potentials of the at least one spine electrode.

2. The apparatus of claim 1, further comprising:
a connector (46) joining the elongated shaft to the end effector, the connector comprising an electrically insulating cover.

3. The apparatus of claim 2, the electrically conducting distal surface of the reference electrode being approximately coplanar or coaxial to a distal end of the connector.

4. The apparatus of any one of claims 2 or 3, the connector being capable of contacting the cardiovascular tissue while the spine electrodes are in contact with the cardiovascular tissue and an outer surface of the reference electrode is electrically insulated, by the connector, from the cardiovascular tissue.

5. The apparatus of any one of claims 2 to 4,
the end effector comprising an unconstrained configuration aligned with a longitudinal axis of the apparatus,
the connector comprising a tubular outer surface comprising a diameter measured orthogonal to the longitudinal axis,
the end effector comprising a height measured orthogonal to the longitudinal axis and a width measured orthogonal to the height and longitudinal axis when the end effector is in the unconstrained configuration,
the height measuring approximately equal to or less than the diameter of the tubular outer surface of the connector, and
the width measuring greater than the diameter of the tubular outer surface of the connector.

6. The apparatus of any one of claims 1 to 5, further comprising:
an irrigation tube (15).

7. The apparatus of any one of claims 1 to 6, the spine electrodes positioned to form a grid when the spine electrodes are configured to contact with the cardiovascular tissue.

8. The apparatus of any one of claims 1 to 7,
the elongated shaft defining a longitudinal axis of the apparatus,
the end effector comprising three loop members each comprising at least one of the spines and joined at a common distal vertex along the longitudinal axis,
each of the three loop members (1,2,3) comprising a respective pair of ends affixed to the distal portion of the elongated shaft, and
the end effector configured to deflect at an angle relative to the longitudinal axis to position the spine electrodes against a planar surface.

9. The apparatus of any one of claims 1 to 8, the spine electrodes being configured to receive electrical potentials from the cardiovascular tissue when in contact with the cardiovascular tissue, or to ablate when in contact with the cardiovascular tissue.

10. A method comprising:
assembling an end effector such that the end effector comprises at least one spine electrode thereon configured to contact cardiovascular tissue and receive electrical potentials from the tissue;
affixing a reference electrode in relation to the end effector such that an electrically conductive distal surface at a distal end of the reference electrode is configured to receive electrical potentials from fluids that act as a referential signal for the electrical potentials received by the electrodes;
affixing the end effector and reference electrode to a distal portion of an elongated shaft such that the reference electrode is disposed in the elongated shaft, the elongated shaft configured to be manipulated at a proximal portion of the elongated shaft to position the distal portion into a heart of a patient.

11. The method of claim 10, further comprising;
joining the elongated shaft to the end effector with a connector; and
electrically insulating a portion of an outer surface of the reference electrode with the connector so that an electrically exposed portion of the outer surface is positioned to preclude the exposed portion from coming into contact with tissue during treatment.

12. The method of claim 11, further comprising:
affixing the reference electrode and the connector such that a distal end of the reference electrode and a distal end of the connector are approximately coplanar or coaxial to each other.

13. The method of any one of claims 11 or 12, further comprising:
joining the elongated shaft to the end effector such that the connector is capable of contacting the cardiovascular tissue while the spine electrode is in contact with the cardiovascular tissue and while the reference electrode is electrically insulated, by the connector, from the cardiovascular tissue.

14. The method of any one of claims 10 to 13, further comprising:
positioning an irrigation tube within the elongated shaft.

15. The apparatus of claim 6 or the method of claim 14, wherein the irrigation tube and the reference electrode protrude from the elongated shaft so that the irrigation tube prevents an outer surface of the reference electrode from coming into contact with the tissue.

## Patentansprüche

1. Vorrichtung, umfassend:
einen länglichen Schaft (9), der einen proximalen Abschnitt (12) und einen distalen Abschnitt (14A) umfasst, wobei der längliche Schaft dazu ausgelegt ist, an dem proximalen Abschnitt manipuliert zu werden, um den distalen Abschnitt in ein Herz eines Patienten zu positionieren;
einen Endeffektor (100), der in der Nähe des distalen Abschnitts des länglichen Schafts angeordnet ist und Spines (1A, 1B, 2A, 2B, 3A, 3B) umfasst, die jeweils mindestens eine Spine-Elektrode (37) tragen, die dazu ausgelegt ist, kardiovaskuläres Gewebe zu kontaktieren und elektrische Potentiale aus dem Gewebe zu empfangen; **gekennzeichnet durch** eine Referenzelektrode (5), die eine elektrisch leitende distale Oberfläche an ihrem distalen Ende umfasst, wobei die Referenzelektrode in dem länglichen Schaft angeordnet ist,
wobei die elektrisch leitende distale Oberfläche dazu ausgelegt ist, elektrische Potentiale aus Fluids zu empfangen, die als ein referentielles Signal für die elektrischen Potentiale der mindestens einen Spine-Elektrode agieren.

2. Vorrichtung nach Anspruch 1, ferner umfassend:
einen Verbinder (46), der den länglichen Schaft mit dem Endeffektor verbindet, wobei der Verbinder eine elektrisch isolierende Abdeckung umfasst.

3. Vorrichtung nach Anspruch 2, wobei die elektrisch leitende distale Oberfläche der Referenzelektrode ungefähr koplanar oder koaxial zu einem distalen Ende des Verbinders ist.

4. Vorrichtung nach einem der Ansprüche 2 oder 3, wobei der Verbinder fähig ist, das kardiovaskuläre Gewebe zu kontaktieren, während die Spine-Elektroden mit dem kardiovaskulären Gewebe in Kontakt sind und eine Außenfläche der Referenzelektrode durch den Verbinder von dem kardiovaskulären Gewebe elektrisch isoliert ist.

5. Vorrichtung nach einem der Ansprüche 2 bis 4,
wobei der Endeffektor eine uneingeschränkte Auslegung umfasst, die auf eine Längsachse der Vorrichtung ausgerichtet ist,
wobei der Verbinder eine röhrenförmige Außenfläche umfasst, die einen Durchmesser umfasst, der orthogonal zur Längsachse gemessen wird,
wobei der Endeffektor eine Höhe umfasst, die orthogonal zur Längsachse gemessen wird, und eine Breite, die orthogonal zur Höhe und zur Längsachse gemessen wird, wenn der Endeffektor in der uneingeschränkten Auslegung ist,
wobei die Höhenmessung ungefähr gleich dem oder geringer als der Durchmesser der röhrenförmigen Außenfläche des Verbinders ist, und
die Breitenmessung größer ist als der Durchmesser der röhrenförmigen Außenfläche des Verbinders.

6. Vorrichtung nach einem der Ansprüche 1 bis 5, ferner umfassend
ein Spülrohr (15).

7. Vorrichtung nach einem der Ansprüche 1 bis 6, wobei die Spine-Elektroden dazu positioniert sind, ein Gitter zu bilden, wenn die Spine-Elektroden dazu ausgelegt sind, das kardiovaskuläre Gewebe zu kontaktieren.

8. Vorrichtung nach einem der Ansprüche 1 bis 7,
wobei der längliche Schaft eine Längsachse der Vorrichtung definiert,
wobei der Endeffektor drei Schleifenelemente umfasst, die jeweils mindestens eines der Spines umfassen und an einem gemeinsamen distalen Scheitelpunkt entlang der Längsachse verbunden sind,
wobei jedes der drei Schleifenelemente (1, 2, 3) ein jeweiliges Paar Enden umfasst, die an dem distalen Abschnitt des länglichen Schafts fixiert sind, und
der Endeffektor dazu ausgelegt ist, in einem Winkel bezogen auf die Längsachse auszulenken, um die Spine-Elektroden gegenüber einer ebenen Fläche zu positionieren.

9. Vorrichtung nach einem der Ansprüche 1 bis 8, wobei die Spine-Elektroden dazu ausgelegt sind, elektrische Potentiale aus dem kardiovaskulären Gewebe zu empfangen, wenn mit dem kardiovaskulären Gewebe in Kontakt, oder zu ablatieren, wenn mit dem kardiovaskulären Gewebe in Kontakt.

10. Verfahren, umfassend:
Montieren eines Endeffektors derart, dass der Endeffektor mindestens eine Spine-Elektrode daran umfasst, die dazu ausgelegt ist, kardiovaskuläres Gewebe zu kontaktieren und elektrische Potentiale von dem Gewebe zu empfangen;
Fixieren einer Referenzelektrode in Bezug auf den Endeffektor, sodass eine elektrisch leitende distale Oberfläche an einem distalen Ende der Referenzelektrode dazu ausgelegt ist, elektrische Potentiale aus den Fluids zu empfangen, die als ein referentielles Signal für die von den Elektroden empfangenen elektrischen Potentiale agieren;
Fixieren des Endeffektors und der Referenzelektrode an einem distalen Abschnitt eines länglichen Schafts, sodass die Referenzelektrode in dem länglichen Schaft angeordnet ist, wobei der längliche Schaft dazu ausgelegt ist, an einem proximalen Abschnitt des länglichen Schafts manipuliert zu werden, um den distalen Abschnitt in ein Herz eines Patienten zu positionieren.

11. Verfahren gemäß Anspruch 10, ferner umfassend:
Verbinden des länglichen Schafts mit dem Endeffektor mit einem Verbinder; und
elektrisches Isolieren eines Abschnitts einer Außenfläche der Referenzelektrode mit dem Verbinder, so dass ein elektrisch freiliegender Abschnitt der Außenfläche positioniert ist, um auszuschließen, dass der freiliegende Abschnitt während der Behandlung mit Gewebe in Kontakt kommt.

12. Verfahren gemäß Anspruch 11, ferner umfassend:
Fixieren der Referenzelektrode und des Verbinders, so dass ein distales Ende der Referenzelektrode und ein distales Ende des Verbinders ungefähr koplanar oder koaxial zueinander sind.

13. Verfahren nach einem der Ansprüche 11 oder 12, ferner umfassend:
Verbinden des länglichen Schafts mit dem Endeffektor, so dass der Verbinder fähig ist, das kardiovaskuläre Gewebe zu kontaktieren, während die Spine-Elektrode mit dem kardiovaskulären Gewebe in Kontakt ist und während die Referenzelektrode durch den Verbinder von dem kardiovaskulären Gewebe elektrisch isoliert ist.

14. Verfahren nach einem der Ansprüche 10 oder 13, ferner umfassend:
Positionieren eines Spülrohrs in dem länglichen Schaft.

15. Vorrichtung nach Anspruch 6 oder Verfahren nach Anspruch 14, wobei das Spülrohr und die Referenzelektrode von dem länglichen Schaft vorstehen, so dass das Spülrohr verhindert, dass eine Außenfläche der Referenzelektrode mit dem Gewebe in Kontakt kommt.

## Revendications

1. Appareil comprenant :
une tige allongée (9) comprenant une partie proximale (12) et une partie distale (14A), la tige allongée étant configurée pour être manipulée au niveau de la partie proximale afin de positionner la partie distale dans le coeur d'un patient ;
un effecteur terminal (100) disposé à proximité de la partie distale de la tige allongée et comprenant des colonnes (1A, 1B, 2A, 2B, 3A, 3B) portant chacune au moins une électrode de colonne (37) configurée pour entrer en contact avec un tissu cardiovasculaire et
recevoir des potentiels électriques du tissu ;
**caractérisé en ce qu'**une électrode de référence (5) comprend une surface distale électriquement conductrice à son extrémité distale, l'électrode de référence étant disposée dans la tige allongée,
la surface distale conductrice d'électricité étant configurée pour recevoir des potentiels électriques de fluides qui agissent comme un signal de référence pour les potentiels électriques de l'au moins une électrode de colonne.

2. Appareil selon la revendication 1, comprenant en outre :
un connecteur (46) reliant la tige allongée à l'effecteur terminal, le connecteur comprenant un couvercle électriquement isolant.

3. Appareil selon la revendication 2, la surface distale électriquement conductrice de l'électrode de référence étant approximativement coplanaire ou coaxiale à une extrémité distale du connecteur.

4. Appareil selon l'une quelconque des revendications 2 ou 3, le connecteur étant capable d'entrer en contact avec le tissu cardiovasculaire alors que les électrodes de colonnes sont en contact avec le tissu cardiovasculaire et qu'une surface extérieure de l'électrode de référence est isolée électriquement, par le connecteur, du tissu cardiovasculaire.

5. Appareil selon l'une quelconque des revendications 2 à 4,
l'effecteur terminal comprenant une configuration non contrainte alignée avec un axe longitudinal de l'appareil,
le connecteur comprenant une surface extérieure tubulaire comprenant un diamètre mesuré orthogonalement à l'axe longitudinal,
l'effecteur terminal comprenant une hauteur mesurée orthogonalement à l'axe longitudinal et une largeur mesurée orthogonalement à la hauteur et à l'axe longitudinal lorsque l'effecteur terminal est dans la configuration non contrainte,
la hauteur étant approximativement égale ou inférieure au diamètre de la surface extérieure tubulaire du connecteur, et
la largeur étant supérieure au diamètre de la surface extérieure tubulaire du connecteur.

6. Appareil selon l'une quelconque des revendications 1 à 5, comprenant en outre :
un tube d'irrigation (15).

7. Appareil selon l'une quelconque des revendications 1 à 6, les électrodes de colonnes étant positionnées pour former une grille lorsque les électrodes de colonnes sont configurées pour entrer en contact avec le tissu cardiovasculaire.

8. Appareil selon l'une quelconque des revendications 1 à 7,
la tige allongée définissant un axe longitudinal de l'appareil,
l'effecteur terminal comprenant trois éléments de boucle comprenant chacun au moins une des colonnes et reliés à un sommet distal commun le long de l'axe longitudinal, chacun des trois éléments de boucle (1, 2, 3) comprenant une paire respective d'extrémités fixées à la partie distale de la tige allongée, et
l'effecteur terminal étant configuré pour dévier à un angle par rapport à l'axe longitudinal afin de positionner les électrodes de colonnes contre une surface plane.

9. Appareil selon l'une quelconque des revendications 1 à 8, les électrodes de colonnes étant configurées pour recevoir des potentiels électriques du tissu cardiovasculaire lorsqu'elles sont en contact avec le tissu cardiovasculaire, ou pour effectuer une ablation lorsqu'elles sont en contact avec le tissu cardiovasculaire.

10. Procédé comprenant :
l'assemblage d'un effecteur terminal de sorte que l'effecteur terminal comprend au moins une électrode de colonne sur celui-ci, configurée pour entrer en contact avec le tissu cardiovasculaire et recevoir des potentiels électriques du tissu ;
la fixation d'une électrode de référence par rapport à l'effecteur terminal de sorte qu'une surface distale électriquement conductrice à une extrémité distale de l'électrode de référence soit configurée pour recevoir des potentiels électriques de fluides qui agissent comme un signal référentiel pour les potentiels électriques reçus par les électrodes ;
la fixation de l'effecteur terminal et de l'électrode de référence à une partie distale d'une tige allongée de sorte que l'électrode de référence soit disposée dans la tige allongée, la tige allongée étant configurée pour être manipulée à une partie proximale de la tige allongée afin de positionner la partie distale dans le coeur d'un patient.

11. Procédé selon la revendication 10, comprenant en outre :
le fait de relier la tige allongée à l'effecteur terminal à l'aide d'un connecteur ; et
l'isolation électrique d'une partie d'une surface extérieure de l'électrode de référence avec le connecteur de sorte qu'une partie électriquement exposée de la surface extérieure soit positionnée de manière à empêcher la partie exposée d'entrer en contact avec le tissu pendant le traitement.

12. Procédé selon la revendication 11, comprenant en outre :
la fixation de l'électrode de référence et du connecteur de telle sorte qu'une extrémité distale de l'électrode de référence et une extrémité distale du connecteur soient approximativement coplanaires ou coaxiales l'une par rapport à l'autre.

13. Procédé selon l'une quelconque des revendications 11 ou 12, comprenant en outre :
le fait de relier la tige allongée à l'effecteur terminal de telle sorte que le connecteur soit capable d'entrer en contact avec le tissu cardiovasculaire alors que l'électrode de colonne est en contact avec le tissu cardiovasculaire et que l'électrode de référence est isolée électriquement, par le connecteur, du tissu cardiovasculaire.

14. Procédé selon l'une quelconque des revendications 10 et 13, comprenant en outre :
le positionnement d'un tube d'irrigation à l'intérieur de la tige allongée.

15. Appareil selon la revendication 6 ou procédé selon la revendication 14, le tube d'irrigation et l'électrode de référence dépassant de la tige allongée de sorte que le tube d'irrigation empêche une surface extérieure de l'électrode de référence d'entrer en contact avec le tissu.
